# FASCICULE DE BREVET EUROPEEN

(11) **EP 1 530 430 B1**
(45) Date de publication et mention de la délivrance du brevet: **31.12.2008**
(21) Numéro de dépôt: 03755645.3
(22) Date de dépôt: 28.07.2003
(51) Int. Cl.: A23L 1/30, C07D 307/36, C11B 7/00

(54) **PROCEDE D'OBTENTION D'UN INSAPONIFIABLE D'AVOCAT RICHE EN LIPIDES FURANIQUES**
GEWINNUNGSVERFAHREN EINER UNVERSEIFBAREN AVOCADOFRAKTION MIT EINEM HÖHEREN FURANLIPIDENGEHALT
METHOD FOR PRODUCING AN AVOCADO UNSAPONIFIABLE RICH IN FURAN LIPIDS

(30) Priorité: 29.07.2002 WO PCT/FR02/02715; 29.07.2002 US 206792
(43) Date de publication de la demande: 18.05.2005
(73) Titulaire: Laboratoires Expanscience, 92400 Courbevoie (FR)
(72) Inventeur: PICCIRILLI, Antoine, F-78000 Versailles (FR); LEGRAND, Jacques, F-61290 Neuilly sur Eure (FR)
(74) Mandataire: Texier, Christian
(86) Numéro de dépôt international: PCT/FR2003/002379
(87) Numéro de publication internationale: WO 2004/012496

(56) Documents cités:
- FR-A- 2 653 974
- FR-A- 2 678 614
- FR-A- 2 678 632
- FR-A- 2 798 667
- US-A- 4 560 568
- FARINES M ET AL: "Influence of Avocado Oil Processing on the Nature of Some Unsaponifiable Constituents" JOURNAL OF THE AMERICAN OIL CHEMISTS' SOCIETY, AMERICAN OIL CHEMISTS' SOCIETY. CHAMPAIGN, US, vol. 72, no. 4, 1995, pages 473-476, XP002144061 ISSN: 0003-021X cité dans la demande
- RANCUREL A: "L'avocat: Son huile et son insaponifiable. Utilisation cosmétique" PARFUMS, COSMETIQUES, AROMES, SOCIETE D'EXPANSION TECHNIQUE ET ECONOMIQUE S.A. PARIS, FR, vol. 61, 1985, pages 91-95, XP002144060 ISSN: 0337-3029

## Description

La présente invention concerne un procédé d'obtention d'un insaponifiable d'avocat riche en lipides furaniques.

L'avocat comprend de manière connue des lipides particuliers de type furanique, dont le principal composant est un furane linoléique :

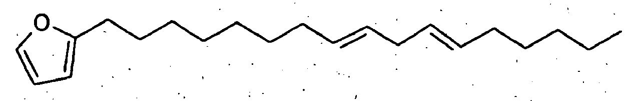

Ainsi, « Par lipides furaniques d'avocat », on entend selon l'invention les composants répondant à la formule : 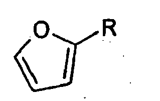 dans laquelle R est une chaîne linéaire hydrocarbonée en C₁₁-C₁₉ de préférence C₁₃-C₁₇ saturée ou comprenant une ou plusieurs insaturations éthyléniques ou acétyléniques. Ces lipides furaniques d'avocat ont été décrits notamment dans Farines, M. et al, 1995, J. of Am. Oil Chem. Soc. 72, 473.

L'insaponifiable est la fraction d'un corps gras qui, après action prolongée d'une base alcaline, reste insoluble dans l'eau et peut être extraite par un solvant organique. Cinq grands groupes de substances sont présents dans la plupart des insaponifiables d'huiles végétales : hydrocarbures saturés ou insaturés, alcools aliphatiques ou terpéniques, stérols, tocophérols, les pigments caroténoïdes et xanthophiles.

Le brevet FR 91 08301 décrit un procédé d'obtention d'un insaponifiable d'avocat à partir d'une huile d'avocat enrichie en l'une de ses fractions, dite H, correspondant en fait à ces mêmes lipides furaniques. La préparation d'un tel insaponifiable riche en lipides furaniques, dont la teneur peut varier de 30 à 60 %, est essentiellement conditionnée à un chauffage contrôlé des fruits frais, préalablement tranchés en fines lamelles, à une température comprise entre 80 et 120°C, et pendant une durée préférentiellement choisie entre 24 à 48 heures. Dans ce procédé, le traitement thermique peut être précédé de la déshydratation du fruit, mais de façon préférée, il est réalisé de façon concomitante au séchage du fruit. Ce traitement thermique permet après extraction, d'obtenir une huile d'avocat riche en lipides furaniques. Enfin, à partir de cette huile, l'obtention de la fraction insaponifiable est effectuée selon un procédé classique de saponification, complété d'une étape d'extraction liquide-liquide.

Ce procédé présente le premier inconvénient de devoir chauffer à une température relativement élevée, au moins égale à 80°C, pendant des temps assez longs, 1 à 2 jours, des lamelles de fruits riches en lipides aisément oxydables dans ces conditions. En outre, ce type de traitement thermique, entraine dans les matières végétales alimentaires, des réactions secondaires de dégradation bien connues de l'homme de métier, telles que les réactions de Maillard, à l'origine du brunissement non voulu des produits, et de l'apparition de flaveurs et d'arômes souvent désagréables.

Par ailleurs, ce type de traitement thermique, effectué sous air, sans inertage préalable, peut s'accompagner de modification chimiques importantes, notamment en présence de substrats thermosensibles tels que les lipides (ex. acides gras insaturés) et leur fraction insaponifiable (ex. vitamine E). Ainsi, les processus thermiques vont favoriser la thermo-oxydation des substrats, les réactions radicalaires responsables de l'apparition de peroxydes, mais aussi de réactions de condensation intra ou intermoléculaires à l'origine de la formation de produits lourds.

De fait, la conjonction de ces processus secondaires non maîtrisés, peut conduire à une altération majeure des caractères organoleptiques des produits traités et à une profonde modification de leurs propriétés physico-chimiques. Enfin, ce type de procédé peut être à l'origine d'une baisse drastique du rendement en produit cible, en l'occurrence, les lipides furaniques, et nuire ainsi à la rentabilité globale du procédé.

Compte tenu de l'intérêt thérapeutique de l'insaponifiable d'avocat riche en lipides furaniques pour son action bénéfique et curative sur le tissu conjonctif, notamment dans les pathologies inflammatoires telles que l'arthrose, les parodontites et la sclérodermie, et de son coût élevé en général, il existe donc un intérêt fort à préparer avec le meilleur rendement possible, des fractions insaponifiables d'huile d'avocat, riches en lipides furaniques et très appauvris en composés d'oxydation et de condensation.

La demanderesse a ainsi mis au point un procédé, permettant d'obtenir avec un rendement élevé, un insaponifiable d'avocat riche en lipides furaniques, à savoir d'une teneur variant de 50 à 80%, présentant de faibles teneurs en produits lourds et en peroxydes.

Ce procédé comprend les étapes successives suivantes :
(1) une étape de déshydratation contrôlée des avocats frais ou ayant subi des transformations préalables, réalisée à une température comprise entre -50°C et 75°C,
(2) une étape d'extraction de l'huile des fruits déshydratés,
(3) une étape, alternativement,
   - a. de traitement thermique de l'huile extraite à une température pouvant varier de 80 à 150°C, éventuellement sous atmosphère inerte, puis d'une étape de concentration de l'huile en sa fraction insaponifiable ou bien,
   - b. d'une étape de concentration de l'huile en sa fraction insaponifiable, puis d'un traitement thermique à une température pouvant varier de 80 à 150°C, éventuellement sous atmosphère inerte, suivie
(4) d'une étape de saponification et d'extraction de l'insaponifiable.
Par « avocat ayant subi des transformations préalables » on entend les co-produits issus des procédés d'extraction des huiles d'avocat frais, notamment ceux issus des procédés dits de centrifugation. Ainsi, on peut notamment citer, à titre d' «avocat ayant subi des transformations préalables », i) les laits d'avocat obtenus par pressage des pulpes, ou encore ii) les produits de débourbage des pulpes partiellement déshuiléés par centrifugation, sous-produits généralement présents en sortie des passoires centrifuges, ou encore les culots de centrifugeuses produits au cours de la séparation.
D'autres sources d'avocat, que l'on fait entrer dans le terme « avocat ayant subi des transformations préalables » peuvent encore être citées : Ainsi, les tourteaux d'avocat, co-produits lors de la pression à froid des fruits (frais ou séchés) ou de l'extraction liquide-solide de l'huile d'avocat de fruits frais ou séchés, à l'aide d'un solvant organique, peuvent aussi constituer en l'état, une matière première alternative utilisable dans le cadre de la présente invention.
Enfin, bien que pauvres en huile, les noyaux d'avocat peuvent potentiellement constituer une source de lipides et être utilisés dans le cadre de la présente invention.

On entend plus généralement par déshydratation, effectuée à l'étape (1) du procédé, l'ensemble des techniques connues de l'homme de métier et qui permettent d'extraire l'eau d'un composé. Parmi ces techniques on peut citer le séchage sous courant d'air chaud ou sous atmosphère contrôlée (ex. azote), à pression atmosphérique ou sous vide, en couche épaisse ou couche mince, mais encore le séchage par micro-ondes, le séchage par pulvérisation, la lyophilisation et la déshydratation osmotique en solution (osmose directe) ou en phase solide (ex. séchage en sacs osmotiques).
Dans le cadre du présent procédé, pour des raisons de facilité de mise en oeuvre industrielle et pour des raisons de coût, le séchage en séchoirs ventilés, en couche mince et sous courant d'air chaud, à une température comprise entre 70 et 75°C, pendant 8 à 36 heures est préféré.

L'étape (2) d'extraction peut être mise en oeuvre par tout moyen connu de l'homme de métier, de préférence par une simple pression à froid ou grâce à un solvant à basse température.

Selon la procédé de l'invention, l'étape de traitement thermique mise en oeuvre à l'étape (3) a. ou b. peut se faire en présence ou non d'un catalyseur acide.
On entend par catalyseurs acides au sens large les catalyseurs minéraux et organiques dits homogènes tels que les acides chlorhydrique, sulfurique, acétique ou paratoluènesulfonique mais aussi, et de préférence, les catalyseurs solides hétérogènes tels que la silice, l'alumine, les silices-alumines, les zircones, les zéolithes; les résines acides. On choisira en particulier les alumines acides de grandes surfaces spécifiques, c'est à dire au moins égales à 200 m²/g.
On préfère pour la mise en oeuvre du procédé de l'invention les catalyseurs de type alumines acides.

Avantageusement, on effectue ce traitement thermique sous un courant continu d'azote.
De préférence, la température de traitement thermique est comprise entre 80 et 130°C.

L'étape de concentration de l'étape (3)a. ou (3)b. peut être une cristallisation à froid ou une distillation moléculaire.
La distillation moléculaire peut être réalisée à une température comprise entre 180 et 260°C en maintenant une pression comprise entre 10⁻³ et 10⁻² mmHg.
Cette étape de distillation moléculaire de l'insaponifiable, est de préférence réalisée en utilisant un dispositif choisi parmi les distillateurs moléculaires de type centrifuge et les dispositifs moléculaires de type à film raclé.
Les distillateurs moléculaires de type centrifuge sont connus de l'homme du métier. Par exemple, la demande EP 493 144 décrit un distillateur moléculaire de ce type. D'une manière générale, le produit à distiller est étalé en couche mince sur la surface chauffée (surface chaude) d'un rotor conique tournant à grande vitesse. L'enceinte de distillation est placée sous vide. Dans ces conditions, il y a évaporation et non pas ébullition, depuis la surface chaude, des constituants de l'insaponifiable, l'avantage étant que l'huile et l'insaponifiable (ces produits étant réputés fragiles) ne sont pas dégradés au cours de l'évaporation.
Les distillateurs moléculaires de type à film raclé, également connus de l'homme du métier, comprennent une chambre de distillation dotée d'un racleur tournant, permettant l'étalement en continu sur la surface d'évaporation (surface chaude) du produit à distiller. Les vapeurs de produit sont condensées par le biais d'un doigt réfrigéré, placé au centre de la chambre de distillation. Les systèmes périphériques d'alimentation et de vide sont très proches de ceux d'un distillateur centrifuge (pompes d'alimentation, pompes à vide à palette et à diffusion d'huile, etc.). La récupération des résidus et des distillats dans des ballons en verre, se fait par écoulement gravitationnel.

L'étape (4) de saponification de l'huile ou de l'extrait huileux peut être mise en oeuvre en présence de potasse ou de soude en milieu alcoolique, de préférence éthanolique, suivie d'une ou plusieurs extraction(s). L'extraction par un solvant organique approprié (extraction liquide-liquide) en vue de séparer les savons d'acides gras et les composés insaponifiables, est particulièrement adaptée. Le solvant organique approprié peut être, par exemple choisi dans le groupe des alcanes, des alcanes halogénés, des solvants aromatiques, des éthers tels que le méthyltertiobutyl ether (MTBE) et de l'éther éthylique, ou tout autre solvant approprié non miscible avec la solution hydro-alcoolique.
De préférence on choisira un alcane halogéné, en particulier le 1,2-dichloroéthane.

La solution d'extraction obtenue est de préférence ensuite centrifugée, filtrée puis lavée à l'eau pour éliminer les traces résiduelles d'alcalinité. Enfin, le solvant d'extraction est évaporé soigneusement pour récupérer l'insaponifiable. On peut également bien entendu prévoir des opérations supplémentaires connues de l'homme du métier, telle qu'une étape de désodorisation.

Les exemples suivants, illustrent l'invention.

A partir d'un même lot d'avocats frais, d'un poids de net de 1000 kg, de la variété Fuerte et d'origine sud-africaine, on constitue 6 sous-lots de 50 kg chacun.

### Exemple 1 / Procédé de traitement thermique poussé du fruit : (non selon l'invention)

50 kg d'avocats frais, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 85°C, sa durée est quant à elle de 48 heures. Une fois séchés, les fruits sont broyés puis soumis à une pression à froid. Cette opération est réalisée sur une petite presse Komet de laboratoire. L'huile extraite est alors filtrée sur büchner, puis stockée sous azote à l'abri de la lumière et de l'humidité.

L'huile obtenue est ensuite distillée dans un distillateur moléculaire à film râclé, de type Leybold KDL 4, à une température de 230°C et sous un vide de 10⁻³ mm de mercure. Le rendement en distillat de cette opération est de 9,2 %.

Dans un réacteur en verre, équipé d'une agitation mécanique et surmonté d'un réfrigérant, le distillat obtenu est saponifié pendant 4 heures, à reflux, en présence de 175 g de potasse à 50% et 875 g d'éthanol. En fin de réaction, le mélange est refroidi à 30°C, puis dilué par ajout d'eau déminéralisée. La solution hydroalcoolique (SHA) obtenue est alors extraite à l'ampoule à décanter par le 1,2-dichloro éthane.

Les phases organiques sont alors rassemblées et séchées sur sulfate de sodium anhydre. La fraction insaponifiable dissoute est enfin récupérée après évaporation du solvant et séchage sous vide. Cette étape est effectuée dans un évaporateur rotatif, à 70°C, sous une vide de 1 mm de mercure, pendant 2 heures. On récupère ainsi 126 g de fraction insaponifiable d'avocat, qui est alors stockée sous azote pour être analysée.

L'analyse physico-chimique et chromatographique de cette fraction a donné les résultats suivants :
- Indice de peroxyde : 87,2 meq O₂/kg
- Indice de saponification : 12,3 mg KOH/g
- Résidu à l'incinération : 0,3 %

- Teneur en lipides furaniques : 49,2 %
- Teneur en alcools gras : 14,7
- Composés lourds : 22,3 %

### Exemple 2 / Procédé de traitement thermique poussé de l'huile :

50 kg d'avocats frais, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 70°C, sa durée est quant à elle de 48 heures. Une fois séchés, les fruits sont broyés puis soumis à une pression à froid. Cette opération est réalisée sur une petite presse Komet de laboratoire. L'huile extraite est alors filtrée sur bûchner, puis stockée sous azote à l'abri de la lumière et de l'humidité.

Cette huile est ensuite chauffée à 85°C, pendant 48 heures, sous un courant continu d'azote, dans un réacteur en verre équipé d'une agitation mécanique.

L'huile obtenue est ensuite distillée dans un distillateur moléculaire à film râclé, de type Leybold KDL 4, à un température de 230°C et sous un vide de 10⁻³ mm de mercure. Le rendement en distillat de cette opération est de 9,4 %.

Dans un réacteur en verre, équipé d'une agitation mécanique et surmonté d'un réfrigérant, 350 g de ce distillat sont saponifiés pendant 4 heures, à reflux, en présence de 175 g de potasse à 50% et 875 g d'éthanol. En fin de réaction, le mélange est refroidi à 30°C, puis dilué par ajout d'eau déminéralisée. La solution hydro-alcoolique (SHA) obtenue est alors extraite à l'ampoule à décanter par le 1,2- dichloro éthane. Les phases organiques sont alors rassemblées et séchées sur sulfate de sodium anhydre. La fraction insaponifiable dissoute est enfin récupérée après évaporation du solvant et séchage sous vide. Cette étape est effectuée dans un évaporateur rotatif, à 70°C, sous un vide de 1 mm de mercure, pendant 2 heures. On récupère ainsi 141 g de fraction insaponifiable d'avocat, qui est alors stockée sous azote pour être analysée.

L'analyse physico-chimique et chromatographique de cette fraction a donné les résultats suivants :
- Indice de peroxyde : 23,2 meq O₂/kg
- Indice de saponification : 11,3 mg KOH/g
- Résidu à l'incinération : 0,2 %

- Teneur en lipides furaniques : 57,6 %
- Teneur en alcools gras : 13,7
- Composés lourds : 14,3 %

### Exemple 3 / Procédé de traitement thermique poussé du distillat :

50 kg d'avocats frais, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 70°C, sa durée est quant à elle de 48 heures. Une fois séchés, les fruits sont broyés puis soumis à une pression à froid. Cette opération est réalisée sur une petite presse Komet de laboratoire. L'huile extraite est alors filtrée sur bûchner, puis stockée sous azote à l'abri de la lumière et de l'humidité.

L'huile obtenue et ensuite distillée dans un distillateur moléculaires à film râclé, de type Leybold KDL 4, à une température de 230°C et sous un vide de 10⁻³ mm de mercure. Le rendement en distillat de cette opération est de 9,9 %.

Ce distillat est ensuite chauffé à 85°C, pendant 48 heures, sous un courant continu d'azote, dans un réacteur en verre équipé d'une agitation mécanique.

Dans un réacteur en verre, équipé d'une agitation mécanique et surmonté d'un réfrigérant, l'huile obtenue est saponifiée pendant 4 heures, à reflux, en présence de 175 g de potasse à 50% et 875 g d'éthanol. En fin de réaction, le mélange est refroidi à 30°C, puis dilué par ajout d'eau déminéralisée. La solution hydro-alcoolique (SHA) obtenue est alors extraite à l'ampoule à décanter par le 1,2-dichloroéthane.

Les phases organiques sont alors rassemblées et séchées sur sulfate de sodium anhydre. La fraction insaponifiable dissoute est enfin récupérée après évaporation du solvant et séchage sous vide. Cette étape est effectuée dans un évaporateur rotatif, à 70°C, sous un vide de 1 mm de mercure, pendant 2 heures. On récupère ainsi 163 g de fraction insaponifiable d'avocat, qui est alors stockée sous azote pour être analysée.
L'analyse physico-chimique et chromatographique de cette fraction a donné les résultats suivants :
- Indice de peroxyde : 26,3 meq O₂/kg
- Indice de saponification : 10,7 mg KOH/g
- Résidu à l'incinération : 0,3 %

- Teneur en lipides furaniques : 62,2 %
- Teneur en alcools gras : 14,1
- Composés lourds : 13,2 %

### Conclusions intermédiaires :

**Tableau 1 : comparaison des différents procédés d'obtention de la fraction insaponifiable d'avocat / Influence de la nature du traitement thermique**

| Exemple | Variante (1) | Gain en Rendement (%) (1) | lipides furaniques (%) (2) | Composés lourds (%) | Indice de peroxyde (meq O₂/kg) |
|---|---|---|---|---|---|
| 1 | Chauffage poussé du fruit | - | 49,2 | 22,3 | 87,2 |
| 2 | Chauffage poussé de l'huile | + 12 % | 57,6 | 14,3 | 23,2 |
| 3 | Chauffage poussé du distillat | + 29 % | 62,2 | 13,2 | 26,3 |

| | | | | | |
|---|---|---|---|---|---|
| (1) Gain par rapport à l'exemple 1 choisi comme procédé de référence (2) Teneur en lipides furaniques de la fraction | | | | | |

insaponifiable obtenue
- Le procédé classique de chauffage poussé du fruit (exemple 1) conduit à un produit fortement oxydé (Ip = 87,2 meq O₂/kg) et présente une teneur notable en composés lourds (22,3 %), composés issus des processus chimiques de condensation, activés par la température et la durée élevée du processus.
- Le procédé de chauffage poussé de l'huile, effectué sous atmosphère inerte, permet un gain notable de rendement (+ 12 % par rapport au procédé de référence de chauffage du fruit). Il conduit aussi à un produit moins oxydé (Indice de péroxyde < 30), moins chargé en composés lourds (environ 14 % contre 22 % précédemment) et inversement plus riche en lipides furaniques (57 % contre 49%).
- Le procédé de chauffage du distillat offre quant à lui un meilleur rendement (+29%) et une forte teneur en lipides furaniques dans l'insaponifiable final (62 %). Par conséquent, le rendement global en lipides furaniques est donc fortement accru.

### Exemple 4 / Procédé de traitement thermique poussé du distillat en présence d'un catalyseur :

50 kg d'avocats frais, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 70°C, sa durée est quant à elle de 48 heures. Une fois séchés, les fruits sont broyés puis soumis à une pression à froid. Cette opération est réalisée sur une petite presse Komet de laboratoire. L'huile extraite est alors filtrée sur bûchner, puis stockée sous azote à l'abri de la lumière et de l'humidité.

L'huile obtenue est ensuite distillée dans un distillateur moléculaire à film râclé, de type Leybold KDL 4, à une température de 230°C et sous un vide de 10⁻³ mm de mercure. Le rendement en distillat de cette opération est de 9,9 %.

Ce distillat est ensuite chauffé à 85°C, pendant 2 heures, en présence de 5% d'alumine acide (catalyseur), sous un courant continu d'azote, et dans un réacteur en verre équipé d'une agitation mécanique.

Dans un réacteur en verre, équipé d'une agitation mécanique et surmonté d'un réfrigérant, le distillat traité thermiquement est filtré et saponifié pendant 4 heures, à reflux, en présence de 175 g de potasse à 50% et 875 g d'éthanol. En fin de réaction, le mélange est refroidi à 30°C, puis dilué par ajout d'eau déminéralisée. La solution hydro-alcoolique (SHA) obtenue est alors extraite à l'ampoule à décanter par le 1,2-dichloroéthane.

Les phases organiques sont alors rassemblées et séchées sur sulfate de sodium anhydre. La fraction insaponifiable dissoute est enfin récupérée après évaporation du solvant et séchage sous vide. Cette étape est effectuée dans un évaporateur rotatif, à 70°C, sous un vide de 1 mm de mercure, pendant 2 heures. On récupère ainsi 126 g de fraction insaponifiable d'avocat, qui est alors stockée sous azote pour être analysée.

L'analyse physico-chimique et chromatographique de cette fraction a donné les résultats suivants :
- Indice de peroxyde : 23,3 meq O₂/kg
- Indice de saponification : 11,3 mg KOH/g
- Résidu à l'incinération : 0,2 %

- Teneur en lipides furaniques : 71,5 %
- Teneur en alcools gras : 13,9
- Composés lourds : 5,2 %

### Exemple 5 / Procédé de traitement thermique poussé du distillat en absence de catalyseur :

50 kg d'avocats frais, sont coupés en fines lamelles de 2 à 5 mm d'épaisseur, noyau compris, à l'aide d'un trancheur à disque. L'outil de séchage est une étuve thermo-régulée à courant d'air chaud. Les avocats tranchés sont répartis sur une épaisseur de 4 à 5 cm sur des clayettes étagées. La température de séchage est fixée à 70°C, sa durée est quant à elle de 48 heures. Une fois séchés, les fruits sont broyés puis soumis à une pression à froid. Cette opération est réalisée sur une petite presse Komet de laboratoire. L'huile extraite est alors filtrée sur büchner, puis stockée sous azote à l'abri de la lumière et de l'humidité.

L'huile obtenue est ensuite distillée dans un distillateur moléculaire à film râclé, de type Leybold KDL 4, à une température de 230°C et sous un vide de 10⁻³ mm de mercure. Le rendement en distillat de cette opération est de 9,9 %.

Ce distillat est ensuite chauffé à 85°C, pendant 2 heure, sous un courant continu d'azote, et dans un réacteur en verre équipé d'une agitation mécanique.

Dans un réacteur en verre, équipé d'une agitation mécanique et surmonté d'un réfrigérant, le distillat traité thermiquement est saponifié pendant 4 heures, à reflux, en présence de 175 g de potasse à 50% et 875 g d'éthanol. En fin de réaction, le mélange est refroidi à 30°C, puis dilué par ajout d'eau déminéralisée. La solution hydro-alcoolique (SHA) obtenue est alors extraite à l'ampoule à décanter par le 1,2-dichloro éthane.

Les phases organiques sont alors rassemblées et séchées sur sulfate de sodium anhydre. La fraction insaponifiable dissoute est enfin récupérée après évaporation du solvant et séchage sous vide. Cette étape est effectuée dans un évaporateur rotatif, à 70°C, sous un vide de 1 mm de mercure, pendant 2 heures. On récupère ainsi 17 g de fraction insaponifiable d'avocat, qui est alors stockée sous azote pour être analysée.

L'analyse physico-chimique et chromatographique de cette fraction a donné les résultats suivants :
- Indice de peroxyde: 21,2 meq O₂/kg
- Indice de saponification : 10,1 mg KOH/g
- Résidu à l'incinération : 0,1 %

- Teneur en lipides furaniques : 70,1 %
- Teneur en alcools gras : 14,2 %
- Composés lourds : 3,2 %

### Conclusion :

L'ajout d'un catalyseur alumine augmente drastiquement la vitesse de la transformation puisque à après 2 heures de traitement thermique on obtient 126 g d'insaponifiable contre à peine 17 g en absence d'alumine.

### Contre-exemple / Procédé sans traitement thermique poussé du fruit ou de son huile optionnellement concentrée :

50 kg d'avocats frais, sont coupés en fines lamelles de 2 à 5 mm d'épaisseur, noyau compris, à l'aide d'un trancheur à disque. L'outil de séchage est une étuve thèrmo-régulée à courant d'air chaud. Les avocats tranchés sont répartis sur une épaisseur de 4 à 5 cm sur des clayettes étagées. La température de séchage est fixée à 65°C, sa durée est quant à elle de 72 heures. Une fois séchés, les fruits sont broyés puis soumis à une pression à froid. Cette opération est réalisée sur une petite presse Komet de laboratoire. L'huile extraite est alors filtrée sur büchner, puis stockée sous azote à l'abri de la lumière et de l'humidité.

L'huile obtenue est ensuite distillée dans un distillateur moléculaire à film râclé, de type Leybold KDL 4, à une température de 230°C et sous un vide de 10⁻³ mm de mercure. Le rendement en distillat de cette opération est de 9,1 %.

Dans un réacteur en verre, équipé d'une agitation mécanique et surmonté d'un réfrigérant, le distillat obtenu est saponifié pendant 4 heures, à reflux, en présence de 175 g de potasse à 50% et 875 g d'éthanol. En fin de réaction, le mélange est refroidi à 30°C, puis dilué par ajout d'eau déminéralisée. La solution hydro-alcoolique (SHA) obtenue est alors extraite à l'ampoule à décanter par le 1,2-dichloroéthane.

Les phases organiques sont alors rassemblées et séchées sur sulfate de sodium anhydre. La fraction insaponifiable dissoute est enfin récupérée après évaporation du solvant sous vide et à basse température. Cette étape est effectuée dans un évaporateur rotatif, à 70°C, sous un vide de 1 mm de mercure, pendant 2 heures. On récupère ainsi 105 g de fraction insaponifiable d'avocat, qui est alors stockée sous azote pour être analysée.

L'analyse physico-chimique et chromatographique de cette fraction a donné les résultats suivants :
- Indice de peroxyde : 5,1 meq O₂/kg
- Indice de saponification : 11,3 mg KOH/g
- Résidu à l'incinération : 0,4 %

- Teneur en lipides furaniques : 5,2 %
- Teneur en alcools gras : 13,7
- Composés lourds : 31,2 %

### Conclusion :

En l'absence d'une étape de chauffage poussé du fruit ou de l'huile qui résulterait de fruits non chauffés, la teneur en lipides furaniques de la fraction insaponifiable d'avocat obtenue est extrêmement faible (largement inférieure à 10%).

## Revendications

1. Procédé d'obtention d'un insaponifiable d'avocat riche en lipides furaniques, **caractérisé en ce qu'**il comprend les étapes successives suivantes :
(1) une étape de déshydratation contrôlée des avocats frais ou ayant subi des transformations préalables, réalisée à une température comprise entre -50°C et 75°C,
(2) une étape d'extraction de l'huile de fruits déshydratés,
(3) une étape, alternativement,
- a. de traitement thermique de l'huile extraite à une température pouvant varier de 80 à 150°C, puis d'une étape de concentration de l'huile en sa fraction insaponifiable ou bien,
- b. d'une étape de concentration de l'huile en sa fraction unsaponifiable, puis d'un traitement thermique à une température pouvant varier de 80 à 150°C,
(4) suivie d'une étape de saponification et d'extraction de l'insaponifiable.

2. Procédé d'obtention d'un insaponifiable d'avocat riche en lipides furaniques selon la revendication 1, **caractérisé en ce que** le traitement thermique lors de l'étape, (3) a. ou (3) b. est effectué en présence d'un catalyseur.

3. Procédé d'obtention d'un insaponifiable d'avocat riche en lipides furaniques selon la revendication 2, **caractérisé en ce que** le catalyseur est un catalyseur acide de type catalyseurs minéraux ou organiques homogènes choisis dans le groupe des acides chlorhydrique, sulfurique, acétique ou paratoluènesulfonique ou bien un catalyseur solide hétérogène choisi dans le groupe constitué par la silice, l'alumine, les silices-alumines, les zircones, les zéolithes et les résines acides.

4. Procédé d'obtention d'un insaponifiable d'avocat riche en lipides furaniques selon la revendication 3, **caractérisé en ce que** le catalyseur est de type alumine acide, de surface spécifique au moins égale à 200 m²/g.

5. Procédé d'obtention d'un insaponifiable d'avocat riche en limpides furaniques selon l'une quelconque des revendications 1 à 4, **caractérisé en ce que** la déshydratation de l'étape (1) est choisie parmi le groupe constitué par le séchage sous courant d'air chaud ou sous atmosphère contrôlée, le séchage à pression atmosphérique ou sous vide, en couche épaisse ou couche mince, le séchage par micro-ondes, le séchage par pulvérisation, la lyophilisation et la déshydratation osmotique en solution ou en phase solide.

6. Procédé d'obtention d'un insaponifiable d'avocat riche en lipides furaniques selon la revendication 5, **caractérisé en ce que** la déshydratation de l'étape (1) consiste en un séchage en séchoirs ventilés, en couche mince et sous courant d'air chaud, à une température comprise entre 70 et 75°C, pendant 8 à 36 heures.

7. Procédé d'obtention d'un insaponifiable d'avocat riche en lipides furaniques selon l'une quelconque des revendications 1 à 6, **caractérisé en ce que** l'extraction de l'étape (2) est effectuée par une simple pression à froid ou grâce à un solvant à basse température.

8. Procédé d'obtention d'un insaponifiable d'avocat riche en lipide furanique selon l'une quelconque des revendications 1 à 7, **caractérisé en ce que** l'étape de concentration de l'étape (3)a. ou (3)b. est une cristallisation à froid ou une distillation moléculaire.

9. Procédé d'obtention d'un insaponifiable d'avocat selon la revendication 8, **caractérisé en ce que** l'étape de concentration est une distillation moléculaire qui est réalisée à une température comprise entre 180 et 260°C en maintenant une pression comprise entre 10⁻³ et 10⁻² mmHg.

10. Procédé d'obtention d'un insaponifiable d'avocat riche en lipides furaniques selon l'une quelconque des revendications 1 à 9, **caractérisé en ce que** la distillation moléculaire est mise en oeuvre dans un dispositif choisi parmi les distillateurs moléculaires de type centrifuge et les dispositifs moléculaires de type à film raclé.

11. Procédé d'obtention d'un insaponifiable d'avocat riche en lipides furaniques selon l'une quelconque des revendications 1 à 10, **caractérisé en ce que** l'étape (4) de saponification de l'huile ou de l'extrait huileux est mise en oeuvre en présence de potasse ou de soude en milieu alcoolique suivie d'une ou plusieurs extraction(s).

12. Procédé d'obtention d'un insaponifiable d'avocat riche en lipides furaniques selon la revendication 11, **caractérisé en ce que** l'extraction a lieu par extraction liquide-liquide avec un solvant organique choisi dans le groupe des alcanes, des alcanes halogènes, des solvants aromatiques et des éthers.

13. Procédé d'obtention d'un insaponifiable d'avocat riche en lipides furaniques selon la revendication 12, **caractérisé en ce que** le solvant organique pour l'extraction est le 1,2-dichloroéthane.

14. Procédé d'obtention d'un insaponifiable d'avocat riche en lipides furaniques selon l'une quelconque des revendications 1 à 13, **caractérisé en ce qu'**il est suivi d'une étape de désodorisation.

## Claims

1. Process for obtaining a furan lipid-rich unsaponifiable material from avocado, **characterized in that** it comprises the following successive steps:
(1) a step of controlled dehydration of fresh avocados or of avocados that have undergone preliminary transformations, performed at a temperature of between -50°C and 75°C,
(2) a step of extraction of the oil from dehydrated fruit,
(3) a step, alternatively,
- a. of heat treatment of the extracted oil at a temperature that can range from 80 to 150°C, and then a step of concentration of the unsaponifiable fraction of the oil, or
- b. of a step of concentration of the unsaponifiable fraction of the oil, followed by a heat treatment at a temperature that can range from 80 to 150°C, followed by
(4) a step of saponification and extraction of the unsaponifiable material.

2. Process for obtaining a furan lipid-rich unsaponifiable material from avocado according to Claim 1, **characterized in that** the heat treatment during step (3) a. or (3) b. is carried out in the presence of a catalyst.

3. Process for obtaining a furan lipid-rich unsaponifiable material from avocado according to Claim 2, **characterized in that** the catalyst is an acid catalyst of homogeneous mineral or organic catalyst type, chosen from the group of hydrochloric acid, sulphuric acid, acetic acid and para-toluenesulphonic acid, or a heterogeneous solid catalyst chosen from the group consisting of silica, alumina, silica-aluminas, zirconias, zeolites and acidic resins.

4. Process for obtaining a furan lipid-rich unsaponifiable material from avocado according to Claim 3, **characterized in that** the catalyst is of acidic alumina type, with a specific surface area at least equal to 200 m²/g.

5. Process for obtaining a furan lipid-rich unsaponifiable material from avocado according to any one of Claims 1 to 4, **characterized in that** the dehydration in step (1) is chosen from the group consisting of drying under a stream of hot air or under a controlled atmosphere, drying at atmospheric pressure or under vacuum, in a thick layer or in a thin layer, microwave drying, spray-drying, freeze-drying and osmotic dehydration in solution or in solid phase.

6. Process for obtaining a furan lipid-rich unsaponifiable material from avocado according to Claim 5, **characterized in that** the dehydration in step (1) consists in drying in ventilated dryers, in a thin layer and under a stream of hot air, at a temperature of between 70 and 75°C for 8 to 36 hours.

7. Process for obtaining a furan lipid-rich unsaponifiable material from avocado according to any one of Claims 1 to 6, **characterized in that** the extraction in step (2) is carried out by a simple cold pressing or by means of a solvent at low temperature.

8. Process for obtaining a furan lipid-rich unsaponifiable material from avocado according to any one of Claims 1 to 7, **characterized in that** the concentration step in step (3)a. or (3)b. is a cold crystallization or a molecular distillation.

9. Process for obtaining an unsaponifiable material from avocado according to Claim 8, **characterized in that** the concentration step is a molecular distillation which is performed at a temperature of between 180 and 260°C while maintaining a pressure of between 10⁻³ and 10⁻² mmHg.

10. Process for obtaining a furan lipid-rich unsaponifiable material from avocado according to any one of Claims 1 to 9, **characterized in that** the molecular distillation is carried out in a device chosen from molecular distillation devices of centrifugal type and molecular devices of scraped-film type.

11. Process for obtaining a furan lipid-rich unsaponifiable material from avocado according to any one of Claims 1 to 10, **characterized in that** step (4) of saponification of the oil or of the oily extract is carried out in the presence of potassium hydroxide or sodium hydroxide in alcoholic medium, followed by one or more extraction(s).

12. Process for obtaining a furan lipid-rich unsaponifiable material from avocado according to Claim 11, **characterized in that** the extraction takes place by liquid-liquid extraction with an organic solvent chosen from the group of alkanes, haloalkanes, aromatic solvents and ethers.

13. Process for obtaining a furan lipid-rich unsaponifiable material from avocado according to Claim 12, **characterized in that** the organic solvent for the extraction is 1,2-dichloroethane.

14. Process for obtaining a furan lipid-rich unsaponifiable material from avocado according to any one of Claims 1 to 13, **characterized in that** it is followed by a deodorization step.

## Patentansprüche

1. Verfahren zur Gewinnung eines an furanischen Lipiden reichen unverseifbaren Anteils von Avocado, **dadurch gekennzeichnet, dass** es die folgenden aufeinander folgenden Schritte umfasst:
(1) einen Schritt einer gesteuerten Dehydratisierung der Avocados, welche frisch sind oder vorab erfolgende Umwandlungen durchlaufen haben, welcher bei einer Temperatur zwischen -50°C und 75°C eingeschlossen ausgeführt wird,
(2) einen Schritt einer Extraktion des Öls der dehydratisierten Früchte,
(3) einen Schritt von alternativ
- a. einer thermischen Behandlung des extrahierten Öls bei einer Temperatur, die von 80 bis 150°C variieren kann, dann eines Schritts einer Aufkonzentrierung des Öls hinsichtlich seines unverseifbaren Anteils oder auch
- b. eines Schritts einer Aufkonzentrierung des Öls hinsichtlich seines unverseifbaren Anteils, dann einer thermischen Behandlung bei einer Temperatur, die von 80 bis 150°C variieren kann,
(4) gefolgt von einem Schritt einer Verseifung und einer Extraktion des unverseifbaren Anteils.

2. Verfahren zur Gewinnung eines an furanischen Lipiden reichen unverseifbaren Anteils von Avocado nach Anspruch 1, **dadurch gekennzeichnet, dass** die thermische Behandlung während des Schritts (3) a. oder (3) b. in Gegenwart eines Katalysators ausgeführt wird.

3. Verfahren zur Gewinnung eines an furanischen Lipiden reichen unverseifbaren Anteils von Avocado nach Anspruch 2, **dadurch gekennzeichnet, dass** der Katalysator ein saurer Katalysator vom Typ mineralischer oder organischer homogener Katalysatoren, welche in der Gruppe von Salzsäure, Schwefelsäure, Essigsäure oder p-Toluolsulfonsäure ausgewählt sind, oder auch ein fester heterogener Katalysator, der in der aus Siliciumdioxid, Aluminiumoxid, den Siliciumdioxid-Aluminiumoxiden, den Zirkoniumdioxiden, den Zeolithen und den sauren Harzen gebildeten Gruppe ausgewählt ist, ist.

4. Verfahren zur Gewinnung eines an furanischen Lipiden reichen unverseifbaren Anteils von Avocado nach Anspruch 3, **dadurch gekennzeichnet, dass** der Katalysator vom Typ eines sauren Aluminiumoxids mit einer spezifischen Oberfläche von mindestens gleich 200 m²/g ist.

5. Verfahren zur Gewinnung eines an furanischen Lipiden reichen unverseifbaren Anteils von Avocado nach einem der Ansprüche 1 bis 4, **dadurch gekennzeichnet, dass** die Dehydratisierung des Schritts (1) in der Gruppe, welche aus der Trocknung unter einem Heißluftstrom oder unter geregelter Atmosphäre, der Trocknung bei Atmosphärendruck oder unter Vakuum, in einer dicken Schicht oder einer dünnen Schicht, der Trocknung durch Mikrowellen, der Trocknung durch Zerstäubung, der Lyophilisation und der osmotischen Dehydratisierung in Lösung oder in fester Phase gebildet wird, ausgewählt ist.

6. Verfahren zur Gewinnung eines an furanischen Lipiden reichen unverseifbaren Anteils von Avocado nach Anspruch 5, **dadurch gekennzeichnet, dass** die Dehydratisierung des Schritts (1) in einer Trocknung in belüfteten Trocknern in einer dünnen Schicht und unter einem Heißluftstrom bei einer Temperatur zwischen 70 und 75°C eingeschlossen während 8 bis 36 h besteht.

7. Verfahren zur Gewinnung eines an furanischen Lipiden reichen unverseifbaren Anteils von Avocado nach einem der Ansprüche 1 bis 6, **dadurch gekennzeichnet, dass** die Extraktion des Schrittes (2) durch ein einfaches Pressen in der Kälte oder dank eines Lösemittels bei niedriger Temperatur ausgeführt wird.

8. Verfahren zur Gewinnung eines an furanischen Lipiden reichen unverseifbaren Anteils von Avocado nach einem der Ansprüche 1 bis 7, **dadurch gekennzeichnet, dass** der Aufkonzentrierungsschritt des Schritts (3)a. oder (3)b. eine Kristallisation in der Kälte oder eine Molekulardestillation ist.

9. Verfahren zur Gewinnung eines unverseifbaren Anteils von Avocado nach Anspruch 8, **dadurch gekennzeichnet, dass** der Aufkonzentrierungsschritt eine Molekulardestillation ist, die bei einer Temperatur zwischen 180°C und 260°C eingeschlossen ausgeführt wird, indem ein Druck zwischen 10⁻³ und 10⁻² mm Hg aufrechterhalten wird.

10. Verfahren zur Gewinnung eines an furanischen Lipiden reichen unverseifbaren Anteils von Avocado nach einem der Ansprüche 1 bis 9, **dadurch gekennzeichnet, dass** die Molekulardestillation in einer Vorrichtung, welche unter den Molekulardestillierapparaten vom Zentrifugentyp und den Molekularvorrichtungen vom Typ derer, die mit einem mittels Rakel abgeschabten Film arbeiten, ausgewählt ist, ausgeführt wird.

11. Verfahren zur Gewinnung eines an furanischen Lipiden reichen unverseifbaren Anteils von Avocado nach einem der Ansprüche 1 bis 10, **dadurch gekennzeichnet, dass** der Schritt (4) einer Verseifung des Öls oder des ölartigen Extrakts in Gegenwart von Ätzkali oder Ätznatron in alkoholischem Milieu ausgeführt wird, gefolgt von einer oder mehreren Extraktion(en).

12. Verfahren zur Gewinnung eines an furanischen Lipiden reichen unverseifbaren Anteils von Avocado nach Anspruch 11, **dadurch gekennzeichnet, dass** die Extraktion durch Flüssig-Flüssig-Extraktion mit einem organischen Lösemittel, das in der Gruppe der Alkane, der halogenierten Alkane, der aromatischen Lösemittel und der Ether ausgewählt ist, erfolgt.

13. Verfahren zur Gewinnung eines an furanischen Lipiden reichen unverseifbaren Anteils von Avocado nach Anspruch 12, **dadurch gekennzeichnet, dass** das organische Lösemittel für die Extraktion 1,2-Dichlorethan ist.

14. Verfahren zur Gewinnung eines an furanischen Lipiden reichen unverseifbaren Anteils von Avocado nach einem der Ansprüche 1 bis 13, **dadurch gekennzeichnet, dass** diesem ein Desodorisierungsschritt folgt.
